# EUROPÄISCHE PATENTSCHRIFT

(11) **EP 3 876 238 B1**
(45) Veröffentlichungstag und Bekanntmachung des Hinweises auf die Patenterteilung: **30.10.2024**
(21) Anmeldenummer: 21159837.0
(22) Anmeldetag: 01.03.2021
(51) Int. Cl.: G16H 20/40

(54) **VERFAHREN UND SYSTEM ZUR UNTERSTÜTZUNG HF- UND/ODER US-CHIRURGISCHER EINGRIFFE SOWIE SOFTWAREPROGRAMMPRODUKT**
METHOD AND SYSTEM FOR SUPPORTING HF- AND / OR US SURGICAL INTERVENTIONS AND SOFTWARE PROGRAM PRODUCT
PROCÉDÉ ET SYSTÈME D'AIDE AUX INTERVENTIONS CHIRURGICALES HF ET/OU US, AINSI QUE PRODUIT PROGRAMME LOGICIEL

(30) Priorität: 04.03.2020 DE 102020105834
(43) Veröffentlichungstag der Anmeldung: 08.09.2021
(73) Patentinhaber: OLYMPUS Winter & Ibe GmbH, 22045 Hamburg (DE)
(72) Erfinder: KRÜGER, Jens, 15732 Eichwalde (DE); JANICH, Fabian, 14469 Potsdam (DE)
(74) Vertreter: Seemann & Partner Patentanwälte mbB

(56) Entgegenhaltungen:
- EP-A1- 3 028 657
- US-A1- 2014 081 659
- US-A1- 2019 206 563

## Beschreibung

Die Erfindung betrifft ein Verfahren und ein System zur Unterstützung HF- und/oder US-chirurgischer Eingriffe, die unter Verwendung einer Mehrzahl von, insbesondere endoskopischen, HF- und/oder US-chirurgischen Instrumenten durchgeführt werden, welche von HF- und/oder US-Generatoren betrieben werden, die HF- und/oder US-Leistung in HF- und/oder US-Modes mit vorbestimmten Betriebsparametersätzen zur Verfügung stellen, sowie ein Computerprogrammprodukt.

Unter endoskopischen Geräten werden im Rahmen der vorliegenden Anmeldung nicht nur Endoskope im engeren Sinne verstanden, sondern allgemein Instrumente, die endoskopartig verwendet werden, beispielsweise, und nicht abschließend, auch Laparoskope.

In der HF-Chirurgie (Hochfrequenz-Chirurgie) werden Eingriffe an Patienten vorgenommen, in denen mittels eines, insbesondere endoskopischen, HF-Instruments eine hochfrequente elektrische Leistung in zu behandelndes Gewebe eingetragen wird, um das Gewebe zu schneiden oder zu koagulieren. Dazu haben die HF-Instrumente monopolare oder bipolare HF-Elektroden an der distalen Spitze ihres Endoskopschafts. Die HF-Leistung wird von HF-Generatoren wie denen der Olympus ESG Generatorfamilie zur Verfügung gestellt, an die die HF-Instrumente angeschlossen werden. In den HF-Generatoren ist eine aufwändige Messtechnik verbaut, um jeden Zustand des Gewebes und des Instruments erfassen zu können, beispielsweise, und nicht abschließend, den Widerstand des Gewebes, Strom- und Spannungsverläufe, die Leistungsabgabe, die Dauer einer Anwendung oder die Anzahl der Aktivierungen. Diese Daten können über eine interne Entwicklungssoftware live an einem Entwicklungsgerät ausgelesen und für die Mode-Entwicklung genutzt werden, also die Entwicklung von Betriebsparametersets, die dann am HF-Generator für bestimmte Anwendungen zur Verfügung stehen und ausgewählt werden können. Die Mode-Entwicklung erfolgt bisher nur in der Entwicklungsphase eines HF-Generators anhand von Labor-Tests, von Versuchstierersatzmodellen oder anhand von Tierversuchen.

Die Entwicklung eines neuen HF-Modes erfolgt über viele Iterationen. Je mehr reale Operationssituationen bekannt sind und im Labor nachgebildet werden können, desto besser kann der HF-Mode auch für den Einsatz durch Anwender optimiert werden. Informationen über die Art und Weise der Anwendung werden über das Produktmanagement, das mit den Anwendern in Kontakt steht, in die Entwicklung eingebracht. Bei dieser Art der Weitergabe werden keine oder nur unzureichende Messwerte übermittelt, es kommt zu Ungenauigkeiten der übergebenen Information, oder zur Auslassung von wesentlichen Informationen. So fehlen beispielsweise häufig detaillierte Angaben über das Verhalten des behandelten Gewebes. Diese unvollständigen oder ungenauen Informationen und Daten führen dazu, dass die Versuchstierersatzmodelle nicht optimiert werden können und auch kein numerisches Modell zur Simulation von Gewebe erstellt werden kann. Dies macht die Entwicklung neuer HF-Modes oder die Verbesserung von vorhandenen HF-Modes sehr aufwendig. Aus diesem Grund wird in der Entwicklung versucht, alle, darunter eventuell auch unnötige, Operationssituationen nachzustellen. Anderseits werden auch Modes optimiert, die in der Praxis vielleicht nicht verwendet werden.

Das Ergebnis dieses Entwicklungsprozesses ist es, dass für chirurgische HF-Eingriffe nicht immer die optimalen HF-Modes, das heißt die optimalen Betriebsparametersätze, an den HF-Generatoren zur Verfügung stehen.

Analog kann auch Ultraschall (US) in endoskopischen oder offen-chirurgischen Anwendungen verwendet werden, um Gewebe zu behandeln. Auch in diesem Gebiet wird der Ultraschall je nach Anwendungsbedarf geregelt erzeugt. Es gibt auch Instrumente, die sowohl HF- als auch US-Leistung zur Verfügung stellen, beispielsweise das Thunderbeat-System der Anmelderin. Die Entwicklung von neuen und verbesserten US-Modes steht vor den gleichen Herausforderungen wie die oben beschriebene Entwicklung von HF-Modes.

Dokument EP 3028657 A1 ist der nächstliegender Stand der Technik und offenbart HF- und US-chirurgischer Geräte, die ihre Betriebsmodi zu aktuellen chirurgischen Umständen anpassen.

Diese Anpassung ist ausschließlich auf lokalen Daten basiert.

Es ist daher die Aufgabe der vorliegenden Erfindung, HF- und/oder US-chirurgische Eingriffe zu verbessern.

Diese Aufgabe wird gelöst durch ein Verfahren zur Unterstützung HF- und/oder US-chirurgischer Eingriffe, die unter Verwendung einer Mehrzahl von, insbesondere endoskopischen, HF- und/oder US-chirurgischen Instrumenten durchgeführt werden, welche von HF- und/oder US-Generatoren betrieben werden, die HF-Leistung in HF-Modes und/oder US-Leistung in US-Modes mit vorbestimmten Betriebsparametersätzen zur Verfügung stellen, mit einer elektrischen Messvorrichtung ausgestattet sind und während der Eingriffe Messungen elektrischer Eigenschaften von behandeltem Gewebe vornehmen, wobei folgende Verfahrensschritte ausgeführt werden:
a) Daten, insbesondere Operationsdaten und/oder Messdaten der Messvorrichtung, werden erfasst und im HF- und/oder US-Generator zwischengespeichert,
b) die zwischengespeicherten Daten werden an eine zentrale Auswertevorrichtung übertragen,
c) die übertragenen Daten werden in der zentralen Auswertevorrichtung gespeichert und ausgewertet und
d) als Ergebnis der Auswertung werden vorhandene HF- und/oder US-Modes angepasst oder verworfen, neue HF-Modes erzeugt, Labortests auf ihre Praxisrelevanz überprüft und gegebenenfalls angepasst und/oder neue Labortests für bislang unbekannte Operationszustände eingeführt.

Die Erfindung beruht auf dem Grundgedanken, dass erstmals Daten von realen Operationen zur Verfügung gestellt werden, um daraus Rückschlüsse auf die Verwendung und die Eignung von HF- und/oder US-Modes von HF- und/oder US-Generatoren zu ziehen und diese gegebenenfalls zu verändern und anzupassen.

Um dies zu ermöglichen, sind mehrere ineinandergreifende Änderungen an den bestehenden Systemen notwendig. Hardwareseitig werden die HF- und/oder US-Generatoren ausgestattet, Daten wie beispielsweise Operationsdaten und Messdaten, zwischenzuspeichern und an eine zentrale Auswertevorrichtung zu übermitteln. Diese Modifikation der HF- und/oder US-Generatoren, die bislang nur mittels eines direkt angeschlossenen Entwicklungsgeräts ausgelesen werden können, sorgt dafür, dass Daten von realen HF- und/oder US-chirurgischen Eingriffen gesammelt und für die Auswertung zur Verfügung gestellt werden. Die zweite Änderung betrifft die Einrichtung einer zentralen Auswertevorrichtung, die die übermittelten Daten von einer Mehrzahl von HF- und/oder US-Generatoren sammelt und auswertet.

Ein Anwendungsbeispiel hierfür ist ein HF-Generatorsystem eines Herstellers, dessen HF-Generatoren mit entsprechenden Funktionalitäten ausgestattet werden, beispielsweise mit einer Zwischenspeichereinheit und einer Übermittlungseinheit. Die verkauften und installierten HF-Generatoren des Herstellers sind so eingerichtet, dass sie ihre Daten, gegebenenfalls anonymisiert, an einen Zentralrechner oder ein zentrales Datenverarbeitungssystem beim Hersteller zurücksenden, das als Auswertevorrichtung arbeitet, so dass der Hersteller Zugriff auf die von den HF-Generatoren übermittelten Daten von den Operationen hat und diese zur Analyse und zur Verbesserung und Veränderung von HF-Modes und gegebenenfalls zur Anpassung der Labortests verwenden kann. Dies gilt analog für ein US-Generatorsystem oder ein hybrides HF- und US-System.

In Ausführungsformen werden im Verfahrensschritt a) als Operationsdaten anonymisierte Patientendaten, insbesondere Erfolgs- und/oder Heilungsdaten, Typ des Eingriffs, Dauer des Eingriffs, eine Bewertung oder Beurteilung des Eingriffs durch einen Anwender und/oder Metadaten der eingesetzten HF- und/oder US-Modes erfasst und zwischengespeichert. Die Bewertung oder Beurteilung des Eingriffs durch einen Anwender, beispielsweise einen Chirurgen, kann in Form einer Beurteilung als "gut" oder "schlecht" erfolgen, oder anhand einer Bewertung des Behandlungserfolgs, ob beispielsweise ein Gefäß erfolgreich versiegelt wurde, was nur anhand der Messdaten alleine teilweise schwer bewertbar ist. Die Metadaten der eingesetzten HF- und/oder US-Modes betreffen beispielsweise die eingestellten Betriebsparameter, die den voreingestellten Betriebsparametersets der HF- und/oder US-Modes entsprechen können, aber auch in Einzelfällen durch den behandelnden Arzt oder eine vor oder während der Operation für die Bedienung des HF- und/oder US-Generators beauftragte Person abgewandelt sein können. Die Metadaten können auch Daten wie Standort, Zeiten, sowie instrumentenspezifische Daten enthalten. Diese Daten sind somit entweder Grunddaten der Operation oder Einstellungen der beteiligten Instrumente, nicht aber Daten, die von einer Messvorrichtung während der Operation gemessen werden. Die in diesem Zusammenhang als Operationsdaten zu erfassenden Daten sind nicht auf die hier genannten Fälle beschränkt und können um geeignete weitere Daten ergänzt werden.

Für veränderliche zu messende Daten werden in Verfahrensschritt a) in Ausführungsformen als Messdaten eine Impedanz oder ein Widerstand des behandelten Gewebes, Strom- und Spannungsverläufe, Leistungsabgabe, Dauer der Anwendung, Anzahl der Aktivierungen, Temperaturen des behandelten Gewebes oder Typen des behandelten Gewebes, insbesondere in zeitlicher Korrelation mit dem angewendeten HF- und/oder US-Mode und/oder der eingebrachten HF- und/oder US-Leistung erfasst und zwischengespeichert. Die in diesem Zusammenhang als Messdaten zu erfassenden Daten sind nicht auf die hier genannten Fälle beschränkt und können um geeignete weitere Daten ergänzt werden.

Die Kombination der Erfassung der Operationsdaten und der Messdaten ist besonders vorteilhaft bei der Auswertung der Eignung der eingesetzten HF- und/oder US-Modes bei realen Operationen.

In Ausführungsformen erfolgt im Verfahrensschritt b) die Übertragung über eine digitale Schnittstelle und Infrastruktur, insbesondere drahtlos. Als digitale Infrastruktur kommen das Internet und auch Lokal Area Networks (LANs) infrage. Eine zumindest teilweise drahtlose Übertragung kann beispielsweise über WLAN oder mobile 4G- oder 5G-Datenverbindungen o.ä. erfolgen.

Im Verfahrensschritt c) erfolgt in Ausführungsformen eine Datenkonsolidierung, eine Extraktion von Daten und/oder Merkmalen, ein Auswerten mittels maschinellen Lernens und/oder ein Auswerten mittels Data Mining. Unter einer Datenkonsolidierung wird im Rahmen der vorliegenden Erfindung verstanden, dass die Daten von den verschiedenen HF-Generatoren, insbesondere wenn es sich um verschiedene Modelle handelt, in ein gemeinsames Datenformat gebracht werden, welches dann Vergleichbarkeit der Daten miteinander herstellt. Bei der Extraktion von Daten und Merkmalen wird beispielsweise auf einem Teil der Daten, die verschiedene Filteranforderungen erfüllen, eine Analyse nur eines Teils der Daten bzw. Merkmale ausgeführt, die für den Zweck der Analyse besonders relevant sind, was die Rechner Anforderungen deutlich begrenzt. Maschinelles Lernen und Datamining kann beispielsweise auf der Grundlage von Behandlungsergebnissen herausfinden, welche Betriebsparametereinstellungen in welchen Situationen zu besonders guten Ergebnissen führen.

Ein Anwendungsbeispiel für Daten und Merkmale, die im Rahmen der Auswertung extrahiert werden können und deren Anwendung ist etwa die gemessene Gewebeimpedanz. Diese kann zu einer automatischen Gewebeerkennung genutzt werden, da verschiedene Gewebetypen verschiedene elektrische Eigenschaften haben. Auch die Regelung der HF-Spannung kann infolge der gemessenen Gewebeimpedanz anzupassen sein, wobei bei einem hochohmigen Arbeitsbereich gegebenenfalls eine höhere Spannung anzusetzen ist. Eine Phasenverschiebung zwischen Strom und Spannung des HF-Signals kann zur Untersuchung des Einflusses von Endoskopen auf das eingesetzte Messsystem verwendet werden und daraufhin die Messgenauigkeit verbessert werden. Mittels Extraktion und Analyse des Verlaufs der Gewebeimpedanz kann die Regelgeschwindigkeit optimiert werden. Bei schnellen Lastsprüngen muss der Regler gegebenenfalls schneller eingestellt werden. Diese Auflistung möglicher Anwendungsfälle ist nicht abschließend.

Beim maschinellen Lernen geht es darum, bestimmte Abhängigkeiten und Merkmale aus einer möglichst großen Anzahl an Trainingsdaten zu extrahieren. Es entsteht dabei ein statistisches Modell dieser Daten. Alternativ können auch Data Mining Ansätze benutzt werden, um neue Zusammenhänge erkennen zu können. Daten können auch in Rohform gespeichert werden, um daraus Merkmale zu gewinnen. Solche Daten können z.B. Log-Informationen des Generators sein, Mitschnitte der Benutzung von der graphischen Benutzerschnittstelle (GUI), Messdaten des Generators wie Leistung, Spannung oder Widerstand, Instrument-Daten oder Aktivierungskanalinformationen, wie beispielsweise ob und wie lange ein Fußschalter gedrückt ist.

Anwendungsbeispiele hierfür umfassen Widerstands- und Leistungsverläufe für bestimmte Operationen, aus denen sich ein statistisches Modell für diese HF-Moden oder das behandelte Gewebe entwickeln lassen, oder Spannungsverläufe der internen Komponenten, aus denen erkennbar ist, ob bei bestimmten HF-Moden oder Instrumenten die Leistungsreserven des Generators optimiert werden können. Aus einem Leistungsabgabeprofil lässt sich die Verwendbarkeit (Usability) von HF-Modes erschließen, etwa wie ein Arzt einen HF-Mode verwendet, ob er mit der Leistungsabgabe klarkommt, ob er öfter stoppt oder neu ansetzen muss. Anhand der Information, ob während einer Operation bestimmte Fehlermeldungen kommen oder es zu ungewünschten Zuständen im Generator kommt, können Verbesserungen der Verwendbarkeit der HF-Modes entwickelt werden, die beispielsweise die Fehlerhäufigkeit reduzieren oder Fehlerschwellen anpassen. Andere Fragestellungen sind, ob es Unterbrechungen im Arbeitsablauf gibt oder ob der Arzt eine bestimmte Einstellung nicht findet und in den Menüs umherkreist. Diese Auswertungen und Fragestellungen können, je nach Anwendung, beispielsweise mit neuronalen Netzen oder klassischen statistischen Methoden durchgeführt werden, da die Fragestellungen wohldefiniert sind.

In Ausführungsformen wird im Verfahrensschritt d) ein HF-Mode angepasst, wenn sich aufgrund der übertragenen Daten mehrerer HF-Generatoren zeigt, dass Betriebsparameter eines eingesetzten HF-Modes für den bestimmungsgemäßen Einsatz zur Verbesserung des Einsatzes anzupassen sind. Dies kann beispielsweise dadurch erkannt werden, dass für bestimmte Typen von Operationen von einigen Ärzten oder anderem Bedienpersonal zunächst ein bestimmter HF-Mode ausgewählt wird, einzelne Betriebsparameter jedoch nach der Auswahl des HF-Modes systematisch verändert werden und sich durch den Vergleich mit anderen Operationen, in denen diese Änderungen nicht stattfinden, herausstellt, dass die Behandlungsergebnisse durch die systematischen Änderungen besser sind. Andererseits kann es sein, dass die abgerufene Leistung oder das abgerufene Leistungsprofil bei bestimmten Operationen nicht derjenigen oder demjenigen entspricht, die oder der bei einem bestimmten HF-Mode eigentlich vorgesehen ist. Beispielsweise könnte bei Verwendung eines schlecht angepassten HF-Modes ein Koagulationssaum erzeugt werden, der aber für das behandelte Gewebe nicht geeignet ist. Hier erfolgt eine Anpassung in die Richtung, dass die bei den realen Operationen gemessenen Leistungen oder Leistungsprofile besser im HF-Mode abgebildet werden. Diese Ausführungen gelten analog für US-Modes.

In weiteren Ausführungsformen wird im Verfahrensschritt d) ein vorhandener HF- und/oder US-Mode verworfen, wenn sich aufgrund der übertragenen Daten mehrerer HF- und/oder US-Generatoren zeigt, dass der HF- und/oder US-Mode nicht oder unterhalb einer vorbestimmten Häufigkeit oder einem vorbestimmten Verwendungsanteil verwendet wird. Weiterhin kann in Ausdrucksformen im Verfahrensschritt d) neue HF- und/oder US-Modes erzeugt werden, wenn sich aufgrund der übertragenen Daten mehrerer HF- und/oder US-Generatoren zeigt, dass die Betriebsparametersätze der vorhandenen HF- und/oder US-Modes für neu identifizierte Einsatzbereiche ungeeignet sind. Entsprechend zu den Änderungen von HF- und/oder US-Modes kann der letztgenannte Fall beispielsweise dadurch identifiziert werden, dass eine Leistung oder ein Leistungsprofil zum Einsatz kommt, das dem eigentlich gewollten Profil des gewählten HF- und/oder US-Modes nicht entspricht, also davon um einen gegebenenfalls vorbestimmten Betrag abweicht.

Diese alternativen oder kumulativ zueinander verwendbaren Optionen führen zu einer Weiterentwicklung der vorbestimmten HF- und/oder US-Modes der HF- und/oder US-Generatoren. Die Änderungen an HF- und/oder US-Modes werden in Ausführungsformen an die vorhandenen HF- und/oder US-Generatoren übermittelt und in den HF-Generatoren umgesetzt und/oder Änderungen an HF- und/oder US-Modes werden bei der Herstellung neuer HF- und/oder US-Generatoren umgesetzt.

In weiteren Ausführungsformen werden im Verfahrensschritt d) Labortests auf ihre Praxisrelevanz überprüft und dann angepasst, wenn sich aufgrund der übertragenen Daten mehrerer HF- und/oder US-Generatoren zeigt, dass die vorhandenen Labortests die in den realen Eingriffen vorkommenden Operationsbedingungen nicht vollständig oder in abgewandelter Form nachbilden. Die Labortests finden üblicherweise an Versuchstieren oder Versuchstierersatzmodellen, also Tieren entnommenen Organen oder Körperteilen, statt. Hierbei handelt es sich um totes, nicht-durchblutetes Gewebe, beispielsweise Rinderherzen. Während der Laborversuche werden die Mode-Parameter beim Schneiden oder Koagulieren live so angepasst, dass der Mode an diesem, passenden Gewebe, optimal funktioniert. Dies wird unter anderem an einem guten Anschnittverhalten, einem definierten Koagulationssaum und einem korrekten Regelverhalten beurteilt. Bei Anwendung im Körper ist die Gewebeimpedanz oftmals eine andere als bei den nicht-durchbluteten Geweben, oder es finden sich noch parasitäre Kapazitäten, die das nicht-durchblutete Gewebe nicht abbilden kann. Aus diesem Grund werden dem Gewebe oftmals passende Lastwiderstände, Kapazitäten und Induktivitäten hinzugeschaltet, um ein möglichst gutes Ersatzmodell zu erhalten. Oftmals reichen diese Maßnahmen nicht aus, um realitätsnahe Ersatzmodelle zu schaffen. Daher werden die Tests gegebenenfalls um eine Durchblutung ergänzt, beispielsweise mittels einer Pumpe, die Schweineblut durch eine Schweineniere pumpt. Auch hierbei werden die Mode-Parameter evaluiert und gegebenenfalls optimiert.

Insgesamt bleibt aber oftmals das Problem, dass die realen Anwendungsszenarien nicht bekannt sind, also unter anderem die Höhe der Gewebeimpedanz, die Schnelligkeit der Änderung der Gewebeimpedanz, oder mit welchen Leistungseinstellungen gearbeitet wird.

Grundsätzlich wird zwar davon ausgegangen, dass die entsprechenden Eingriffe an den Versuchstieren oder Versuchstierersatzmodellen im Labor die realen Eingriffe beim Menschen in zufriedenstellender Weise simulieren. Dies ist aber aus den zuvor genannten Gründen nicht immer der Fall. Wenn die Messdaten von den HF-Generatoren den Rückschluss zulassen, dass die tatsächlichen Vorgänge und Leistungsentnahmen bei Operationen an Menschen von den Laborbedingungen abweichen, können die Tests so verändert werden, dass sie sich den gemessenen Bedingungen annähern. Dies kann auch dazu führen, dass eine kleinere Anzahl von Versuchstieren für die Entwicklung von HF- und/oder US-Modes benötigt wird.

Weiterhin werden in Ausführungsformen im Verfahrensschritt d) neue Labortests für bislang unbekannte Operationszustände eingeführt, wenn sich aufgrund der übertragenen Daten mehrerer HF- und/oder US-Generatoren zeigt, dass die HF- und/oder US-Generatoren Operationszustände erfassen, die in den Labortests nicht vorkommen. Dies vervollständigt die Datenbasis, die für die Entwicklung von HF- und/oder US-Modes zur Verfügung steht und verbessert somit die mit dem entsprechend ausgestatteten HF- und/oder US-Generatoren erzielten Ergebnisse.

Die der Erfindung zugrunde liegende Aufgabe wird auch durch ein System zur Unterstützung HF- und/oder US-chirurgischer Eingriffe gelöst, umfassend eine Mehrzahl von, insbesondere endoskopischen, HF- und/oder US-chirurgischen Instrumenten, eine Mehrzahl von HF- und/oder US-Generatoren, und wenigstens eine zentrale Auswertevorrichtung, wobei die HF- und/oder US-Generatoren ausgebildet sind, jeweils ein oder mehrere der HF- und/oder US-chirurgischen Instrumente zu betreiben und HF- und/oder US-Leistung in HF- und/oder US-Modes mit vorbestimmten Betriebsparametersätzen zur Verfügung zu stellen, wobei die HF- und/oder US-Generatoren jeweils mit einer elektrischen Messvorrichtung ausgestattet sind, um während der Eingriffe Messungen elektrischer Eigenschaften von behandeltem Gewebe vorzunehmen, dadurch gekennzeichnet, dass die HF- und/oder US-Generatoren ausgebildet und eingerichtet sind, Daten zwischenzuspeichern und an die wenigstens eine zentrale Auswertevorrichtung zu übermitteln, wobei die wenigstens eine zentrale Auswertevorrichtung ausgebildet und eingerichtet ist, die übertragenen Daten zu speichern und auszuwerten und als Ergebnis der Auswertung vorhandene HF- und/oder US-Modes anzupassen oder zu verwerfen, neue HF- und/oder US-Modes zu erzeugen, Labortests auf ihre Praxisrelevanz zu überprüfen und gegebenenfalls anzupassen und/oder neue Labortests für bislang unbekannte Operationszustände einzuführen. Vorzugsweise ist die zentrale Auswertevorrichtung ausgebildet und eingerichtet, die Verfahrensschritte c) und d) eines zuvor beschriebenen erfindungsgemäßen Verfahrens auszuführen.

Das System bezieht sich auf das erfindungsgemäße Verfahren und verwirklicht somit auch dessen Merkmale, Eigenschaften und Vorteile.

Die der Erfindung zugrundeliegende Aufgabe wird auch durch ein Computerprogrammprodukt mit Programmcodemitteln gelöst, die ausgebildet sind, die Verfahrensschritte c) und d) eines zuvor beschriebenen erfindungsgemäßen Verfahrens auszuführen, wenn es auf einer Auswertevorrichtung eines zuvor beschriebenen erfindungsgemäßen Systems abläuft. Auch das Computerprogrammprodukt verwirklicht somit die Merkmale, Eigenschaften und Vorteile des erfindungsgemäßen Verfahrens.

Weitere Merkmale der Erfindung werden aus der Beschreibung erfindungsgemäßer Ausführungsformen zusammen mit den Ansprüchen und der beigefügten Zeichnung ersichtlich. Erfindungsgemäße Ausführungsformen können einzelne Merkmale oder eine Kombination mehrerer Merkmale erfüllen.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnung beschrieben, wobei bezüglich aller im Text nicht näher erläuterten erfindungsgemäßen Einzelheiten ausdrücklich auf die Zeichnung verwiesen wird. Es zeigt:

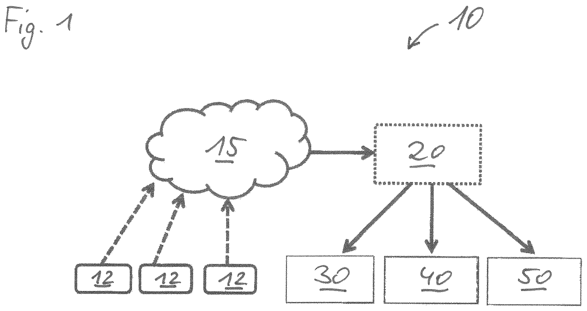

- Fig. 1: eine schematische Darstellung eines erfindungsgemäßen Systems.

Die folgende Beschreibung basiert auf einem HF-System, ist jedoch analog ohne Einschränkungen auf ein Ultraschallsystem oder ein hybrides HF/US-System übertragbar.

Figur 1 zeigt schematisch eine mögliche Konfiguration eines erfindungsgemäßen Systems 10, welches eine Mehrzahl von HF-Generatoren 12 umfasst, die erfindungsgemäß ausgestattet sind, Operationsdaten und/oder Messdaten zwischenzuspeichern und an eine zentrale Auswertevorrichtung 15, welche eine Datenbank für Operationsanwendungen aufweist, zu übermitteln. Die Übermittlung kann über das Internet passieren und lokal entweder kabelgebunden oder über WLAN oder auch über das mobile 4G-Netz oder 5G-Netz oder andere geeignete Kommunikationsnetze und Kommunikationsmittel erfolgen.

Die verschiedenen HF-Generatoren 12, die an verschiedenen Standorten lokalisiert sein können, beispielsweise in verschiedenen Städten oder Ländern, in Kliniken und klinikunabhängigen Arztpraxen, werden bei einer Vielzahl von HF- und/oder US-chirurgischen Eingriffen unter einer Vielzahl von verschiedenen Bedingungen und mit einer Vielzahl von verschiedenen Betriebsparameterkombinationen bzw. HF-Modes verwendet. Mit ihrer Messelektronik zeichnen die HF-Generatoren 12 während der Operation verschiedene Messgrößen auf, beispielsweise die eingebrachte Leistung, den Widerstand des behandelten Gewebes usw. Ebenfalls können die HF-Generatoren 12 mit einem Operationsmanagementsystem eines Krankenhauses verbunden sein und Daten über den Patienten und den geplanten/durchgeführten Eingriff erhalten und ebenfalls an die Auswertevorrichtung 15, gegebenenfalls in anonymisierter Form, übermitteln.

Durch die Vielzahl von verbundenen HF-Generatoren 12 und die Vielzahl der damit durchgeführten Eingriffe steht der Auswertevorrichtung 15 eine große und ständig wachsende Datenbasis zur Verfügung, anhand derer überprüft werden kann, ob die vorhandenen HF-Modes den Anforderungen der Praxis entsprechen, überhaupt angewählt werden, und es kann festgestellt werden, ob die Laborversuche, die für die Entwicklung von HF-Modes verwendet werden, überhaupt die Realität in ausreichender Weise abbilden. Die Auswertung 20 findet in der Auswertevorrichtung 15 mithilfe von Algorithmen statt, die verschiedenartig aufgebaut sein können, beispielsweise in Form von der Erkennung von Häufungen von Betriebsparametern in der Praxis und Vergleich mit vorhandenen Voreinstellungen, Vergleich der Messdaten mit angenommenen optimalen Messdaten, also beruhend auf einer statistischen Analyse, gegebenenfalls verbunden mit maschinellem Lernen, bei dem beispielsweise ein neuronales Netz anhand von Trainingsdaten trainiert wird, günstige und ungünstige Behandlungsverläufe zu erkennen.

Die Auswertung 20 kann zu verschiedenen Resultaten führen. Ein typisches Resultat ist eine HF-Mode-Entwicklung 30, also das Erkennen, dass an den vorhandenen HF-Modes Verbesserungen notwendig sind, dass bestimmte HF-Modes überflüssig sind oder dass neue HF-Modes geschaffen werden müssen. Das zur Verfügung stellen von verbesserten HF-Modes verbessert und unterstützt die HF- und/oder US-chirurgischen Eingriffe vom Augenblick der Einführung der neuen verbesserten HF-Modes an.

Eine weitere Konsequenz der Auswertung 20 kann eine Testbench 40 für HF-Modes sein. Die im realen Einsatz gesammelten Daten können bei einer virtuellen Testbench beispielsweise dazu dienen, die aufgezeichneten Operationen mit gegenüber den realen zugrundeliegenden Eingriffen veränderten Betriebsparametern zu simulieren und Vorhersagen über das Ergebnis der Eingriffe als Resultat der Veränderungen der Betriebsparameter der HF-Generatoren 12 zu treffen. Eine solche Vorgehensweise erspart eine große Anzahl an ansonsten notwendigen Tierversuchen.

Weiterhin können auch Laborversuche selbst angepasst werden, um den Verlauf tatsächlicher HF- und/oder US-chirurgischer Eingriffe besser zu simulieren als bislang möglich. Laborversuche, die nach Vergleich mit den realen Daten Verläufe oder Operationsbedingungen beschreiben, die in der Praxis nicht oder nur kaum vorkommen, können gestrichen werden, was ebenfalls den Einsatz von Versuchstieren reduzieren kann.

Schließlich können auch Marktdaten 50 aus der Untersuchung 20 extrahiert werden, um beispielsweise eine bessere Platzierung oder Erreichbarkeit häufig genutzter HF-Modes in den Bedienungsmenüs der HF-Generatoren 12 zu erreichen und somit das System für Anwender besser bedienbar zu machen. Hierunter fällt auch die Erkenntnis, ob HF-Modes entfallen können, die nicht oder kaum genutzt werden.

### Bezugszeichenliste

- 10: System
- 12: HF-Generator
- 15: Auswertevorrichtung mit Datenbank für OP-Anwendungen
- 20: Auswertung
- 30: HF-Mode-Entwicklung
- 40: Testbench für HF-Modes
- 50: Marktdaten

## Patentansprüche

1. Verfahren zur Unterstützung HF- und/oder US-chirurgischer Eingriffe, die unter Verwendung einer Mehrzahl von, insbesondere endoskopischen, HF- und/oder US-chirurgischen Instrumenten durchgeführt werden, welche von HF- und/oder US-Generatoren (12) betrieben werden, die HF- und/oder US-Leistung in HF- und/oder US-Modes mit vorbestimmten Betriebsparametersätzen zur Verfügung stellen, mit einer elektrischen Messvorrichtung ausgestattet sind und während der Eingriffe Messungen elektrischer Eigenschaften von behandeltem Gewebe vornehmen, wobei folgende Verfahrensschritte ausgeführt werden:
a) Daten, insbesondere Operationsdaten und/oder Messdaten der Messvorrichtung, werden erfasst und im HF- und/oder US-Generator (12) zwischengespeichert,
**dadurch gekennzeichnet, dass** die weiteren folgenden Verfahrensschritte ausgeführt werden:
b) die zwischengespeicherten Daten werden an eine zentrale Auswertevorrichtung (15) übertragen,
c) die übertragenen Daten werden in der zentralen Auswertevorrichtung (15) gespeichert und ausgewertet und
d) als Ergebnis der Auswertung werden vorhandene HF- und/oder US-Modes angepasst oder verworfen, neue HF- und/oder US-Modes erzeugt, Labortests auf ihre Praxisrelevanz überprüft und gegebenenfalls angepasst und/oder neue Labortests für bislang unbekannte Operationszustände eingeführt.

2. Verfahren nach Anspruch 1, **dadurch gekennzeichnet, dass** im Verfahrensschritt a) als Operationsdaten anonymisierte Patientendaten, insbesondere Erfolgs- und/oder Heilungsdaten, Typ des Eingriffs, Dauer des Eingriffs, eine Bewertung oder Beurteilung des Eingriffs durch einen Anwender und/oder Metadaten der eingesetzten HF- und/oder US-Modes erfasst und zwischengespeichert werden.

3. Verfahren nach Anspruch 1 oder 2, **dadurch gekennzeichnet, dass** im Verfahrensschritt a) als Messdaten eine Impedanz oder ein Widerstand des behandelten Gewebes, Strom- und Spannungsverläufe, Leistungsabgabe, Dauer der Anwendung, Anzahl der Aktivierungen, Temperaturen des behandelten Gewebes oder Typen des behandelten Gewebes, insbesondere in zeitlicher Korrelation mit dem angewendeten HF- und/oder US-Mode und/oder der eingebrachten HF- und/oder US-Leistung erfasst und zwischengespeichert werden.

4. Verfahren nach einem der Ansprüche 1 bis 3, **dadurch gekennzeichnet, dass** im Verfahrensschritt b) die Übertragung über eine digitale Schnittstelle und Infrastruktur erfolgt, insbesondere drahtlos.

5. Verfahren nach einem der Ansprüche 1 bis 4, **dadurch gekennzeichnet, dass** im Verfahrensschritt c) eine Datenkonsolidierung, eine Extraktion von Daten und/oder Merkmalen, ein Auswerten mittels maschinellen Lernens und/oder ein Auswerten mittels Data Mining erfolgt.

6. Verfahren nach einem der Ansprüche 1 bis 5, **dadurch gekennzeichnet, dass** im Verfahrensschritt d) ein HF- und/oder US-Mode angepasst wird, wenn sich aufgrund der übertragenen Daten mehrerer HF- und/oder US-Generatoren (12) zeigt, dass Betriebsparameter eines eingesetzten HF- und/oder US-Modes für den bestimmungsgemäßen Einsatz zur Verbesserung des Einsatzes anzupassen sind.

7. Verfahren nach einem der Ansprüche 1 bis 6, **dadurch gekennzeichnet, dass** im Verfahrensschritt d) ein vorhandener HF- und/oder US-Mode verworfen wird, wenn sich aufgrund der übertragenen Daten mehrerer HF- und/oder US-Generatoren (12) zeigt, dass der HF- und/oder US-Mode nicht oder unterhalb einer vorbestimmten Häufigkeit oder einem vorbestimmten Verwendungsanteil verwendet wird.

8. Verfahren nach einem der Ansprüche 1 bis 7, **dadurch gekennzeichnet, dass** im Verfahrensschritt d) neue HF- und/oder US-Modes erzeugt werden, wenn sich aufgrund der übertragenen Daten mehrerer HF- und/oder US-Generatoren (12) zeigt, dass die Betriebsparametersätze der vorhandenen HF- und/oder US-Modes für neu identifizierte Einsatzbereiche ungeeignet sind.

9. Verfahren nach einem der Ansprüche 6 bis 8, **dadurch gekennzeichnet, dass** Änderungen an HF- und/oder US-Modes an die vorhandenen HF- und/oder US-Generatoren (12) übermittelt werden und in den HF- und/oder US-Generatoren (12) umgesetzt werden und/oder Änderungen an HF- und/oder US-Modes bei der Herstellung neuer HF- und/oder US-Generatoren (12) umgesetzt werden.

10. Verfahren nach einem der Ansprüche 1 bis 9, **dadurch gekennzeichnet, dass** im Verfahrensschritt d) Labortests auf ihre Praxisrelevanz überprüft werden und dann angepasst werden, wenn sich aufgrund der übertragenen Daten mehrerer HF- und/oder US-Generatoren (12) zeigt, dass die vorhandenen Labortests die in den realen Eingriffen vorkommenden Operationsbedingungen nicht vollständig oder in abgewandelter Form nachbilden.

11. Verfahren nach einem der Ansprüche 1 bis 10, **dadurch gekennzeichnet, dass** im Verfahrensschritt d) neue Labortests für bislang unbekannte Operationszustände eingeführt werden, wenn sich aufgrund der übertragenen Daten mehrerer HF- und/oder US-Generatoren (12) zeigt, dass die HF- und/oder US-Generatoren (12) Operationszustände erfassen, die in den Labortests nicht vorkommen.

12. System (10) zur Unterstützung HF- und/oder US-chirurgischer Eingriffe, umfassend eine Mehrzahl von, insbesondere endoskopischen, HF- und/oder US-chirurgischen Instrumenten, eine Mehrzahl von HF- und/oder US-Generatoren (12) und wenigstens eine zentrale Auswertevorrichtung (15), wobei die HF- und/oder US-Generatoren (12) ausgebildet sind, jeweils eins oder mehrere der HF- und/oder US-chirurgischen Instrumente zu betreiben und HF- und/oder US-Leistung in HF- und/oder US-Modes mit vorbestimmten Betriebsparametersätzen zur Verfügung zu stellen, wobei die HF- und/oder US-Generatoren (12) jeweils mit einer elektrischen Messvorrichtung ausgestattet sind, um während der Eingriffe Messungen elektrischer Eigenschaften von behandeltem Gewebe vorzunehmen, **dadurch gekennzeichnet, dass** die HF- und/oder US-Generatoren (12) ausgebildet und eingerichtet sind, Daten zwischenzuspeichern und an die wenigstens eine zentrale Auswertevorrichtung (15) zu übermitteln, wobei die wenigstens eine zentrale Auswertevorrichtung (15) ausgebildet und eingerichtet ist, die übertragenen Daten zu speichern und auszuwerten und als Ergebnis der Auswertung vorhandene HF- und/oder US-Modes anzupassen oder zu verwerfen, neue HF- und/oder US-Modes zu erzeugen, Labortests auf ihre Praxisrelevanz zu überprüfen und gegebenenfalls anzupassen und/oder neue Labortests für bislang unbekannte Operationszustände einzuführen.

13. System (10) nach Anspruch 12, **dadurch gekennzeichnet, dass** die zentrale Auswertevorrichtung (15) ausgebildet und eingerichtet ist, die Verfahrensschritte c) und d) eines Verfahrens gemäß einem der Ansprüche 1 bis 11 auszuführen.

14. Computerprogrammprodukt mit Programmcodemitteln, die ausgebildet sind, die Verfahrensschritte c) und d) eines Verfahrens nach einem der Ansprüche 1 bis 11 auszuführen, wenn es auf einer Auswertevorrichtung eines Systems (10) nach Anspruch 12 oder 13 abläuft.

## Claims

1. A method for supporting HF and/or US surgical procedures which are carried out using a plurality of, in particular endoscopic, HF and/or US surgical instruments, which are operated by HF and/or US generators (12) that supply HF and/or US power in HF and/or US modes with predetermined operating parameter sets, are equipped with an electrical measuring device and carry out measurements of electrical properties of treated tissue during the procedures, wherein the following method steps are performed:
a) data, in particular surgical data and/or measurement data of the measuring device, are recorded and temporarily stored in the HF and/or US generator (12),
**characterized in that** the following further method steps are performed:
b) the temporarily stored data are transferred to a central analysis device (15),
c) the transferred data are stored and analyzed in the central analysis device (15), and,
d) as a result of the analysis, existing HF and/or US modes are adapted or rejected, new HF and/or US modes are generated, laboratory tests are checked for their relevance to practical application and adapted as needed, and/or new laboratory tests are introduced for previously unknown surgical states.

2. The method according to claim 1, **characterized in that** anonymized patient data, in particular success and/or recovery data, type of procedure, duration of the procedure, an evaluation or assessment of the procedure by a user and/or metadata of the HF and/or US modes used, are recorded and temporarily stored as surgical data in method step a).

3. The method according to claim 1 or 2, **characterized in that** an impedance or a resistance of the treated tissue, current and voltage characteristics, power output, duration of the application, number of activations, temperatures of the treated tissue, or types of the treated tissue, in particular in a time correlation with the used HF and/or US mode and/or the HF and/or US power introduced are recorded and temporarily stored as measurement data in method step a).

4. The method according to one of claims 1 to 3, **characterized in that** the transfer in method step b) takes place via a digital, in particular wireless, interface and infrastructure.

5. The method according to one of claims 1 to 4, **characterized in that** a data consolidation, an extraction of data and/or features, an analysis by means of machine learning and/or an analysis by means of data mining take place in method step c).

6. The method according to one of claims 1 to 5, **characterized in that** an HF and/or US mode is adapted in method step d) if, due to the transferred data of several HF and/or US generators (12), it is found that operating parameters of an HF and/or US mode used must be adapted for the intended use to improve the use.

7. The method according to one of claims 1 to 6, **characterized in that** an existing HF and/or US mode is rejected in method step d) if it is found based on the transferred data of several HF and/or US generators (12) that the HF and/or US mode is not used or is used with less than a predetermined frequency or a predetermined usage share.

8. The method according to one of claims 1 to 7, **characterized in that** new HF and/or US modes are generated in method step d) if, due to the transferred data of several HF and/or US generators (12), it is found that the operating parameter sets of the existing HF and/or US modes are not suitable for newly identified fields of use.

9. The method according to one of claims 6 to 8, **characterized in that** changes in HF and/or US modes are transmitted to the existing HF and/or US generators (12) and implemented in the HF and/or US generators (12) and/or changes in HF and/or US modes are implemented when new HF and/or US generators (12) are manufactured.

10. The method according to one of claims 1 to 9, **characterized in that**, in method step d), laboratory tests are checked for their relevance to practical applications and are then adapted if, due to the transferred data of several HF and/or US generators (12), it is found that the existing laboratory tests do not replicate the surgical conditions occurring during the real procedures fully or replicate them in a modified form.

11. The method according to one of claims 1 to 10, **characterized in that**, in method step d), new laboratory tests for thus far unknown surgical states are introduced if, due to the transferred data of several HF and/or US generators (12), it is found that the HF and/or US generators (12) record surgical states that do not occur in the laboratory tests.

12. A system (10) for supporting HF and/or US surgical procedures, comprising a plurality of, in particular endoscopic, HF and/or US surgical instruments, a plurality of HF and/or US generators (12), and at least one central analysis device (15), wherein the HF and/or US generators (12) are designed to each operate one or more of the HF and/or US surgical instruments and supply HF and/or US power in HF and/or US modes with predetermined operating parameter sets, wherein the HF and/or US generators (12) are each equipped with an electrical measuring device in order to perform measurements of electrical properties of treated tissue during the procedures, **characterized in that** the HF and/or US generators (12) are designed and configured to temporarily store data and transmit them to the at least one central analysis device (15), wherein the at least one central analysis device (15) is designed and configured to store and analyze the transferred data and, as a result of the analysis, adapt or reject existing HF and/or US modes, generate new HF and/or US modes, check laboratory tests for their relevance to practical application and adapt them if necessary, and/or to introduce new laboratory tests for thus far unknown surgical states.

13. The system according to claim 12, **characterized in that** the central analysis device (15) is designed and configured to perform the method steps c) and d) of a method according to one of claims 1 to 11.

14. A software program product with program code means that are designed to execute the method steps c) and d) of a method according to one of claims 1 to 11 when it is run in an analysis device of a system (10) according to one of claims 12 or 13.

## Revendications

1. Procédé pour assister des interventions chirurgicales HF (en allemand « Hochfrequenz », hautes fréquences) et/ou US (en allemand « Ultraschall », ultrasons), qui sont mises en oeuvre en utilisant une pluralité d'instruments chirurgicaux HF et/ou US, en particulier endoscopiques, qui sont alimentés au moyen de générateurs à HF et/ou à US (12), qui fournissent une alimentation HF et/ou US dans des modes HF et/ou US avec des ensembles de paramètres de fonctionnement prédéterminés, qui sont équipés d'un dispositif de mesure électrique et qui effectuent des mesures de propriétés électriques de tissus traités pendant les interventions, les étapes de procédé suivantes étant mises en oeuvre :
a) des données, en particulier des données d'opération et/ou des données de mesure du dispositif de mesure, sont saisies et stockées temporairement dans le générateur HF et/ou US (12),
**caractérisé en ce que** les autres étapes de procédé suivantes sont mises en oeuvre :
b) les données stockées temporairement sont transmises à un dispositif d'évaluation central (15),
c) les données transmises sont mémorisées et évaluées dans le dispositif central d'évaluation (15), et
d) en tant que résultat de l'évaluation, les modes HF et/ou US existants sont adaptés ou rejetés, de nouveaux modes HF et/ou US sont générés, des tests de laboratoire sont vérifiés quant à leur pertinence pratique et éventuellement adaptés et/ou de nouveaux tests de laboratoire sont introduits pour des états opératoires inconnus jusqu'à présent.

2. Procédé selon la revendication 1, **caractérisé en ce que**, dans l'étape de procédé a), des données de patient anonymisées, en particulier des données de succès et/ou de guérison, le type d'intervention, la durée de l'intervention, une évaluation ou une appréciation de l'intervention par un utilisateur et/ou des métadonnées des modes HF et/ou US utilisés sont enregistrées et stockées temporairement comme données d'opération.

3. Procédé selon la revendication 1 ou la revendication 2, **caractérisé en ce que**, dans l'étape a) du procédé, en tant que données de mesure, une impédance ou une résistance du tissu traité, des courbes de courant et de tension, une puissance délivrée, la durée de l'application, le nombre d'activations, des températures du tissu traité ou des types du tissu traité, en particulier en corrélation temporelle avec le mode HF et/ou US appliqué et/ou la puissance HF et/ou US introduite, sont enregistrés et mémorisés temporairement.

4. Procédé selon l'une des revendications 1 à 3, **caractérisé en ce que** dans l'étape b) du procédé, la transmission s'effectue via une interface et une infrastructure numériques, notamment sans fil.

5. Procédé selon l'une des revendications 1 à 4, **caractérisé en ce que**, dans l'étape c) du procédé, on procède à une consolidation de données, à une extraction de données et/ou de caractéristiques, à une évaluation au moyen d'un apprentissage automatique et/ou à une évaluation au moyen d'une exploration de données.

6. Procédé selon l'une des revendications 1 à 5, **caractérisé en ce que** dans l'étape d) du procédé, un mode HF et/ou US est adapté s'il s'avère, sur la base des données transmises de plusieurs générateurs HF et/ou US (12), que des paramètres de fonctionnement d'un mode HF et/ou US utilisé doivent être adaptés pour l'utilisation conforme à la destination afin d'améliorer l'utilisation.

7. Procédé selon l'une des revendications 1 à 6, **caractérisé en ce que** dans l'étape d) du procédé, un mode HF et/ou US existant est rejeté s'il s'avère, sur la base des données transmises de plusieurs générateurs HF et/ou US (12), que le mode HF et/ou US n'est pas utilisé ou est utilisé à une fréquence inférieure à une fréquence prédéterminée ou à un pourcentage d'utilisation prédéterminé.

8. Procédé selon l'une des revendications 1 à 7, **caractérisé en ce qu'**à l'étape d) du procédé, de nouveaux modes HF et/ou US sont générés s'il s'avère, sur la base des données transmises de plusieurs générateurs HF et/ou US (12), que les ensembles de paramètres de fonctionnement des modes HF et/ou US existants ne sont pas adaptés à des domaines d'utilisation nouvellement identifiés.

9. Procédé selon l'une des revendications 6 à 8, **caractérisé en ce que** les modifications des modes HF et/ou US sont transmises aux générateurs HF et/ou US (12) existants et sont mises en oeuvre dans les générateurs HF et/ou US (12) et/ou les modifications des modes HF et/ou US sont mises en oeuvre lors de la fabrication de nouveaux générateurs HF et/ou US (12).

10. Procédé selon l'une des revendications 1 à 9, **caractérisé en ce que**, dans l'étape d) du procédé, des tests de laboratoire sont vérifiés quant à leur pertinence pratique et sont ensuite adaptés s'il s'avère, sur la base des données transmises de plusieurs générateurs HF et/ou US (12), que les tests de laboratoire existants ne reproduisent pas entièrement ou sous une forme modifiée les conditions opératoires rencontrées dans les interventions réelles.

11. Procédé selon l'une des revendications 1 à 10, **caractérisé en ce que**, à l'étape d) du procédé, de nouveaux tests de laboratoire sont introduits pour des conditions opératoires inconnues jusqu'à présent s'il s'avère, sur la base des données transmises de plusieurs générateurs HF et/ou US (12), que les générateurs HF et/ou US (12) détectent des conditions opératoires qui n'apparaissent pas dans les tests de laboratoire.

12. Système (10) pour assister des interventions chirurgicales HF (en allemand « Hochfrequenz », hautes fréquences) et/ou US (en allemand « Ultraschall », ultrasons), comprenant une pluralité d'instruments chirurgicaux HF et/ou US, en particulier endoscopiques, une pluralité de générateurs HF et/ou US (12) et au moins un dispositif d'évaluation central (15), les générateurs HF et/ou US (12) étant conçus de façon à exploiter respectivement un ou plusieurs des instruments chirurgicaux HF et/ou US et de mettre à disposition une alimentation HF et/ou US dans des modes HF et/ou US avec des ensembles de paramètres de fonctionnement prédéterminés, les générateurs HF et/ou US (12) étant équipés respectivement d'un dispositif de mesure électrique, pour effectuer des mesures de propriétés électriques de tissu traité pendant les interventions, **caractérisé en ce que** les générateurs HF et/ou US (12) sont conçus et agencés pour mémoriser temporairement des données et les transmettre à l'au moins un dispositif d'évaluation central (15), l'au moins un dispositif d'évaluation central (15) étant conçu et agencé de façon à mémoriser et évaluer les données transmises et, en tant que résultat de l'évaluation, à adapter ou rejeter les modes HF et/ou US existants, de générer de nouveaux modes HF et/ou US, de vérifier la pertinence pratique des tests de laboratoire et de les adapter le cas échéant et/ou d'introduire de nouveaux tests de laboratoire pour des états opératoires inconnus jusqu'à présent.

13. Système (10) selon la revendication 12, **caractérisé en ce que** le dispositif central d'évaluation (15) est conçu et agencé pour exécuter les étapes c) et d) d'un procédé selon l'une des revendications 1 à 11.

14. Produit de programme d'ordinateur comprenant des moyens de code de programme configurés pour exécuter les étapes c) et d) d'un procédé selon l'une des revendications 1 à 11, lorsqu'il est exécuté sur un dispositif d'évaluation d'un système (10) selon la revendication 12 ou la revendication 13.
